(19) Europäisches Patentamt
European Patent Office
Office européen des brevets

(11) **EP 1 137 405 B1**

(12) **EUROPÄISCHE PATENTSCHRIFT**

(45) Veröffentlichungstag und Bekanntmachung des Hinweises auf die Patenterteilung:
**31.03.2010 Patentblatt 2010/13**

(51) Int Cl.:
*A61K 9/70* $^{(2006.01)}$    *A61K 9/20* $^{(2006.01)}$
*A61K 31/7056* $^{(2006.01)}$    *A61K 31/7036* $^{(2006.01)}$

(21) Anmeldenummer: **99966929.4**

(22) Anmeldetag: **08.12.1999**

(86) Internationale Anmeldenummer:
**PCT/EP1999/009633**

(87) Internationale Veröffentlichungsnummer:
**WO 2000/033822 (15.06.2000 Gazette 2000/24)**

(54) **WIRKSTOFFMATRIX IN FORM EINES PORÖSEN VLIESES AUS KOLLAGENFIBRILLEN, VERFAHREN ZU IHRER HERSTELLUNG UND VERWENDUNG**

ACTIVE INGREDIENT MATRIX IN THE FORM OF A BIOLOGICALLY RESORBABLE POROUS NON-WOVEN MADE OF COLLAGEN FIBRILS, METHOD FOR THE PRODUCTION AND USE THEREOF

MATRICE DE PRINCIPES ACTIFS SOUS FORME D'UN NON-TISSE POREUX RESORBABLE BIOLOGIQUEMENT, SON PROCEDE DE PRODUCTION ET SON UTILISATION

(84) Benannte Vertragsstaaten:
**DE ES FR GB IT**

(30) Priorität: **09.12.1998 DE 19856668**

(43) Veröffentlichungstag der Anmeldung:
**04.10.2001 Patentblatt 2001/40**

(73) Patentinhaber: **Aesculap AG**
**78532 Tuttlingen (DE)**

(72) Erfinder: **SCHOLL, Edmund**
**D-34212 Melsungen (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
**EP-A- 0 137 297    EP-A- 0 170 979**
**EP-A- 0 173 186    DE-A- 4 404 018**
**DE-B- 2 730 623**

• **WACHOL-DREWEK Z, ET AL.: "Comparative investigation of drug delivery of collagen implants saturated in antibiotic solutions and a sponge containing gentamycin" BIOMATERIALS, Bd. 17, Nr. 17, 1996, Seiten 1733-1738, XP004032983 ISSN 0142-9612**

**EP 1 137 405 B1**

**Beschreibung**

Die Erfindung betrifft eine Wirkstoffmatrix in Form eines biologisch resorbierbaren porösen Vlieses aus Kollagenfibrillen in lyophilisierter Form; ein Verfahren zur Herstellung der Wirkstoffmatrix und ihre Verwendung.

Der Bedarf an resorbierbaren Hämostypika hat in den vergangenen Jahren zur Entwicklung einer Reihe von Produkten auf Kollagenbasis geführt. Kollagenschwämme werden seit vielen Jahren in großem Umfang klinisch eingesetzt. Als Beispiel für den chirurgischen Einsatz seien genannt:

- kapillare Blutungen
- parenchymatöse Hämorrhagien
- unterstützende Maßnahmen für andere hämostypische Techniken.

Kollagenschwämme in Kombination mit den kommerziell erhältlichen Fibrinklebesystemen werden zur stillung diffuser Blutungen, vor allem im Bereich der parenchymatösen Organe, verwendet.

Seit einigen Jahren liegen Produkte im Handel vor, die aus Kollagen beladen mit Aminoglycosiden bestehen. Bei diesen Produkten handelt es sich um gefriergetrocknete Lösungen aus gelöstem Kollagen und Gentamicinsulfat. Der Nachteil dieser gefriergetrockneten antibiotikahaltigen Kollagenschwämme besteht darin, daß nach Implantation die Wirkung des Wirkstoffes schnell nachläßt.

In der Patentschrift DE 32 12 412 C2 wird eine gewebeverklebbare kollagene Wundauflage beschrieben. Isolierte Rindersehnen werden homogenisiert und anschließend in zitronensaurer Lösung bzw. essigsaurer Pepsinlösung das lösliche Kollagen extrahiert. Das so extrahierte, gelöste Kollagen wird nach Dialyse mit entsprechenden Wirkstoffen wie Antifibrinolytika bzw. wasserlöslichen Antibiotika versetzt und gefriergetrocknet.

Die Offenlegungsschrift DE 31 24 981 A1 beschreibt eine wirkstoffhaltige Kollageneinlage zum Einführen in Knochen oder Weichteile. Auch hier wird das Kollagen als zitronensaurer Extrakt aus Rindersehnen gewonnen und dieser Extrakt mit dem Wirkstoff versehen. Der Extrakt, bestehend aus gelöstem Kollagen und gelöstem Antibiotikum wird lyophilisiert. Wird diese Kollageneinlage zum Beispiel in die Tibia eingeführt, so wird sie dort innerhalb von 3 Wochen vollständig resorbiert, wobei während dieser Zeit Antibiotikum freigegeben wird.

In der europäischen Patentschrift EP 0 360 180 B1 wird ein Verfahren zur Herstellung von Kollagenschäumen in Form endloser Bänder beschrieben. Auch hier wird das Kollagen in Lösung gebracht, in dem ein definierter Teil der intermolekularen Bindungen gespalten wird. Diese Spaltung wird so geführt, dass ein Großteil des Kollagens wasserlöslich gemacht wird. Die intramolekularen Bindungen des Kollagens werden nicht angegriffen Die Spaltung kann aber auch so gesteuert werden, dass nur eine geringe Anzahl intermolekularer Bindungen gespalten wird, so dass überwiegend höhermolekulare, wasserunlösliche Kollagenaggregate erhalten werden, die in Wasser dispergiert sind. Mit welchen Hilfsmitteln die kontrollierte intermolekulare Spaltung durchgeführt wird, ist nicht beschrieben.

In der europäischen Patentanmeldung EP0137297 A werden resorbierbare Wirkstoffdepots auf Basis von Kollagen beschrieben. Hier wird das Kollagen, das als Vlies oder Schwammstruktur ausgebildet ist, mit einem Antibiotikum sowie mit einem polyanionischen bioresorbierbaren Polymer beladen.

In der Publikation von Wachol-Drewek, Pfeiffer und Scholl (Biomaterials, Bd. 17 (1996), 1733-1738) werden ebenfalls Kollagenimplantate verschiedenster Struktur sowie Gelatineschwämme beschrieben, die als Depot für Antibiotika eingesetzt werden. Die Implantate als auch die Schwämme werden mit wässrigen Antibiotikalösungen getränkt und anschließend die Dauer der Wirkstoffabgabe dokumentiert. Für Gentamycinsulfat konnte die längste Dauer im Vergleich zu anderen Antibiotika festgestellt werden. Hier lag die Zeitspanne der Abgabe zwischen 3 und 4 Tagen.

Weitere Verfahren zur Herstellung von Kollagenschwämmen durch Gefriertrocknung von Kollagenlösungen oder-dispersionen sind seit langem bekannt (z. B. EP 0 209 726, Beutler/Eblingerl Lindner ; DE-OS 32 03 957, Eckmayer ; US-PS 3 157 524, Artandi ; DE-OS 33 15 678, Cioca und DE 18 11 290, Chvapil).

Es besteht der Wunsch, eine Wirkstoffmatrix zu schaffen, bei der die Wirkung des Wirkstoffes über längere Zeit anhält.

Weiterhin soll die Wirkstoffmatrix in einfacher Weise herstellbar sein, biologisch verträglich sein und Wirkstoffe bzw. Wirkstoffkombinationen unterschiedlicher Art aufnehmen können.

Erfindungsgemäß wird dies gelöst durch eine Wirkstoffmatrix mit den Merkmalen der unabhängigen Ansprüche 1 und 2. Bevorzugte Ausführungsformen sind Gegenstand der abhängigen Ansprüche 3 bis 10. Weiterhin betrifft die Erfindung ein Verfahren mit den Merkmalen des unabhängigen Anspruchs 11. Bevorzugte Ausführungsformen sind Gegenstand der abhängigen Ansprüche 12 bis 21. Die Verwendung der Wirkstoffmatrix wird im unabhängigen Anspruch 22 beschrieben.

Gegenstand der Erfindung ist eine Wirkstoffmatrix in Form eines biologisch resorbierbaren porösen Vlieses aus unvernetzten Kollagenfibrillen in lyophilisierter Form mit einer retardierten Freigabe von Wirkstoffen über mehr als 10 Tage und enthaltend in homogener Verteilung 3 bis 30 Gew.% mindestens eines in Wasser und Körperflüssigkeiten schwerlöslichen Wirkstoffs, wobei mindestens ein Wirkstoff in physiologischem Milieu eine Löslichkeit von < 10 mg/mL aufweist, wobei die Wirkstoffmatrix eine offene Struktur aufweist, Nassstabilität besitzt und außer den Kollagenfibrillen als Trägerstruktur und dem mindestens einen Wirkstoff im Wesentlichen frei von weiteren Bestandteilen ist und im Wesentlichen salzfrei ist, und worin der mindestens eine Wirkstoff nach Implantation retardiert und in geeigneter Konzentration freigesetzt wird.

Desweiteren ist Gegenstand der Erfindung eine biologisch abbaubare Wirkstoffmatrix in Form eines offenporigen Vlieses oder Schwammes aus unvernetzten resorbierbaren Kollagenfibrillen, insbesondere zur Herstellung der Wirkstoffmatrix nach Anspruch 1, dadurch erhältlich, dass man Stücke aus gereinigter, entfetteter und getrockneter Haut in verdünnten wässrigen Lösungen organischer Säuren quellen lässt, bis ein elastisches Material entsteht, die gequollenen Stücke unter Vergrößerung des pH-Wertes mehrfach mit wässrigen Medien, insbesondere entmineralisiertem Wasser, spült, die gespülten Stücke zur Bildung einer Suspension von Kollagenfibrillen mechanisch zerfasert, die eine pH-Wert von 3,5 bis < 4,8 aufweisende, gießfähige Kollagensuspension mit mindestens einem Wirkstoff in feinverteilter Form versetzt und homogenisiert, wobei mindestens ein Wirkstofff in physiologischem Milieu schwerlöslich ist und eine Löslichkeit < 10 mg/mL aufweist, und die wirkstoffhaltige Suspension anschließend zu dem Vlies bzw. Schwamm lyophilisiert.

Die erfndungsgemässen Wirkstoffmatrizen, die als wirkstoffhaltige Kollagene implantatgeeignet sind, erfüllen folgende Anforderungen :

Die Wirkstoff bzw. Trägermatrix ist sterilisierbar, resorbierbar, zeigt keine bleibende Gewebereaktion, bringt keine Störung der Wundheilung, ist leicht applizierbar und bietet einen großflächige Gewebekontakt.

Die Wirkstoff-Freisetzung ist protrahiert, sie ist vollständig. Es lassen sich hohe lokale Gewebespiegel erreichen, verbunden mit einer geringen systemischen Konzentration.

Durch die Erfindung wird weiterhin ein Verfahren geschaffen, das die Einarbeitung von in Wasser schwerlöslichen Wirkstoffformen, insbesondere Arzneiformen, in eine Kollagensuspension ermöglicht. Die Einarbeitung erfolgt in der Weise, dass die Homogenität der Wirkstoffverteüung gewährleistet ist.

Gegenstand der Erfindung ist ein Verfahren zur Herstellung einer biologisch abbaubaren Wirkstoffmatrix in Form eines offenporigen Vlieses aus unvernetzten resorbierbaren Kollagenfibrillen, insbesondere bovinen Ursprungs, das dadurch gekennzeichnet ist, dass man gereinigte, entfettete und getrocknete Hautstücke in verdünnten organischen Säuren quellen lässt, bis ein elastisches Material entsteht, die gequollenen Stücke unter Vergrösserung des pH-Wertes mehrfach mit wässrigen Medien spült, die gequollene Stücke zur Bildung einer Suspension von Kollagenfibrillen mechanisch zerfasert, die einen pH-Wert von > 3,5 bis < 4,8 aufweisende gießfähige Kollagensuspension mit mindestens einem schwerlöslichen Wirkstoff in fein verteilter Form versetzt und homogenisiert und die wirkstoffhaltige Suspension anschließend zu dem Vlies lyophilisiert.

Als Ausgangsmaterial für die Wirkstoffmatrix dient vorzugsweise Rinderhaut. Zur Erzielung der quellbaren Stücke wird die Haut vorzugsweise nach Äscherung und Entfernung der Epidermis und Subcutis zu würfelartigen Stücken zerkleinert. Diese werden dann anschliessend gereinigt, entfettet und getrocknet, so dass sie in einer für die Quellung geeigneten Form vorliegen.

Die erfindungsgemässe Wirkstoffmatrix liegt in Vorteil mit einer Schichtdicke von 0,5 mm bis 15 mm, insbesondere 2 mm bis 5 mm, vor. Im unteren dünnen Bereich kann man von einem Vlies sprechen. Im oberen dicken Bereich kommt die Struktur der Wirkstoffmatrix einem Schwamm nahe und kann als solcher bezeichnet werden. Die Dichte des Vlieses liegt in der Regel zwischen 12 mg/cm$^3$ und 180 mg/cm$^3$, insbesondere 40 mg/cm$^3$ und 80 mg/cm$^3$. Je höher die Dichte ist, desto größer ist die Resorptionszeit, was auch einen Einfluss auf die Dauer der Wirkstoffabgabe hat.

Aufgrund seiner Herstellung durch Lyophilisation kann das Porenvolumen der erfindungsgemäßen Wirkstoffmatrix sehr hoch gehalten werden. In der Regel liegt es bei 60 bis 80 % des Gesamtvolumens. Die mittlere Porengröße liegt etwa im Bereich von 20 bis 150 $\mu$m. Die spezifische Oberfläche, gemessen nach Brunauer, Emmett und Teller (BET) liegt in der Regel bei 150 bis 350 m$^2$/m$^2$ Kollagenvlies. Dies bedingt auch eine hohe bevorzugte Luftdurchlässigkeit von 2.500 bis 5.000 ml/cm$^2$/min, insbesondere 2.700 bis 3.400 ml/cm$^2$/min, bei einer Schichtdicke von 4,2 mm.

Als in Körperflüssigkeiten schwerlösliche Wirkstoffe gelten solche, die im physiologischen Milieu eine Löslichkeit von kleiner 10 mg/ml besitzen, besonders wichtig und vorteilhaft sind diejenigen Wirkstoffe, die eine Löslichkeit von gleich oder kleiner 1 mg/ml haben. Wirkstoffe können sowohl Arzneimittelfunktion haben, was in der Regel der Fall ist. Es können aber auch andere Substanzen als Wirkstoffe eingesetzt werden, wie beispielsweise Diagnostika, bei denen es genauso wichtig sein kann, dass sie über einen grösseren Zeitraum langsam freigesetzt werden.

Als geeignete Arzneimittel seien hier genannt:

Aus der Klasse der Steroid-Antibiotika : Fusidinsäure.
Aus der Klasse der Sulfonamide : Silber-Sulfadiazin
Makrolid-Antibiotika : Erythromycin, Rifampicin.
Aus der Klasse der Aminoglycosid-Antibiotika : Clindamycin Palmitat, Clindamycin-Palmitat-Hydrochlorid (Sobelin$^R$, Fa. Pharmacia et Upjohn) sowie Gentamycin-Crobefat (E. Merck, Darmstadt, EMD 46/217, EP 0 173 186 A1, Beispiel 9).

Aus der Klasse der Glycopeptide : Vancomycin.

Aus der Klasse der Chinolone : Nalidixinsäure, Ciprofloxacin.

Der Gehalt an Wirkstoff in der Wirkstoffmatrix liegt zwischen 3 und 30 Gew.%, bezogen auf das Gesamtgewicht der lyophilisierten Wirkstoffmatrix, insbesondere zwischen 5 und 20 Gew.%. Das Flächengewicht der Wirkstoffmatrix kann je nach Schichtdicke in weiten Grenzen variieren. In der Regel liegt es zwischen 1 und 50 mg/cm$^2$, insbesondere zwischen 10 und 20 mg/cm$^2$.

Unter den Arzneimitteln mit antibiotischen Eigenschaften sind die Aminoglycosid-Antibiotika Clindamycin-Palmitat bzw. Clindamycin-Palmitat-Hydrochlorid sowie Gentamycin-Crobefat besonders bevorzugt. Die erfindungsgemäße Wirkstoffmatrix kann auch mehrere schwerlösliche Wirkstoffe, insbesondere mit unterschiedlicher Wirkungsrichtung, enthalten. In ähnlicher Weise ist auch möglich, neben dem mindestens einen schwerlöslichen Wirkstoff einen oder mehrere Wirkstoffe mit weniger schwerer Löslichkeit oder leichter Löslichkeit in der Wirkstoffmatrix vorzusehen. Auch hier ist es möglich, Wirkstoffe mit unterschiedlicher Wirkungsrichtung vorzusehen. In der Regel ist es bevorzugt, Wirkstoffe mit gleicher Wirkungsrichtung aber unterschiedlich schneller Freigabe vorzusehen. So kann beispielsweise bei den Antibiotika ein Antibiotikum mit retardierter Freigabe wie Gentamycin-Crobefat kombiniert sein mit einem Antibiotikum gleicher Richtungswirkung mit schneller Freigabe, wie Gentamycin-Sulfat. Dadurch ist es möglich, erwünschte anfängliche hohe Gewebespiegel an Antibiotikum zu erzielen, verbunden mit einer lang andauernden retardierten Freigabe des schwerlöslichen Antibiotikums. Die erfindungsgemäße Wirkstoffmatrix eignet sich deshalb in hervorragender Weise zur Verwendung als implantierbares und resorbierbares Depot für Wirkstoffe mit retardierter Wirkstoffabgabe, ggf. in Zusammenhang mit Wirkstoffen mit rascher Wirkstoffabgabe.

Bei der Herstellung der erfindungsgemäßen Wirkstoffmatrix wurde gefunden, daß sich die Handhabung der Suspension von Kollagenfibrillen, die in der Suspension in weitgehend isolierter Form vorliegen, wesentlich vereinfachen läßt, wenn die Suspension einen pH-Wert von > 3,5 bis < 4,8 besitzt. In diesem pH-Bereich ist die Kollagensuspension gießfähig und ermöglicht es, die Wirkstoffe nach ihrer Zugabe in homogener Weise in das Suspension zu verteilen, so daß sie in der fertigen Matrix ebenfalls in homogener Form, eingebettet zwischen die Kollagenfibrillen, vorliegen. Es ist grundsätzlich möglich, den geeigneten pH-Wert nach der mechanischen Zerfaserung der Kollagenstruktur durch Zugabe von Säure oder Base einzustellen. Mit großem Vorteil für Verfahren und Produkt verbunden ist jedoch eine bevorzugte Arbeitsweise, bei der die Säurekonzentration bereits beim Quellvorgang so eingestellt wird, daß nach dem Spülen der gequollenen Kollagenstücke und beim anschließenden Zerfasern der Stücke in die Kollagenfibrillen eine suspension erhalten wird, bei der sich dieser pH-Bereich von selbst ergibt. Ein pH-Bereich zwischen 4,0 und 4,5 ist bevorzugt. Da der pH-Bereich durch Wahl der Konzentration der organischen Säure in Kombination mit der Anzahl der Spülungen und ggf. der Menge der Spülflüssigkeit, einstellbar ist, bestehen hier genügend Variationsmöglichkeiten, um das gewünschte Ergebnis zu erhalten. Normalerweise werden mindestens zwei Spülungen vorgenommen, insbesondere mindestens fünf Spülungen. Bei einem Spülzyklus wird nach jeder Spülung das Spülwasser entfernt. In der Regel wird mit entmineralisiertem Wasser gespült bzw. gewaschen. Wird der Wirkstoff in wässrigem Milieu zugegeben, beispielsweise in einer Suspension, dann besitzt diese vorzugsweise in etwa den gleichen pH-Wert, um pH-Verschiebungen durch die Wirkstoffzugabe zu vermeiden. Dadurch, daß zum Spülen bzw. Waschen entmineralisiertes Wasser verwendet wird und sich eine pH-Korrektur nach dem Spülen erübrigt, läßt sich der Eintrag von Salzen in die Wirkstoffmatrix vermeiden, wodurch die Wirkstoffmatrix im wesentlichen salzfrei ist, was in vielen Fällen erwünscht ist.

Die Quellung kann während einer Zeit von 5 bis 60 Stunden, insbesondere zwischen 6 und 48 Stunden, vorgenommen werden. Die Quelldauer hängt von der Säurekonzentration und vom Ursprung des Kollagenmaterials ab. Die Konzentration der organischen Säure beim Quellvorgang beträgt normalerweise 0,01 bis 2 N, insbesondere 0,05 bis 0,5 N, wobei in der Regel mit 0,1 N gearbeitet wird. Eine geeignete organische Säure ist Essigsäure. Auch andere organische Säuren können verwendet werden, wobei solche mit biologischer Verträglichkeit bevorzugt sind. Flüchtige Säuren sind besonders bevorzugt, weil sie bei der Lyophilisation entfernt werden.

Durch die Quellung werden die Hautstücke, die, wie oben erwähnt, insbesondere bovinen Ursprungs sind, auf das 3 bis 10fache, insbesondere 4 bis 8fache, ihres Gewichtes aufgequollen. Dabei wird die Größe der vorgereinigten trockenen Hautstücke mit Vorteil so ausgewählt, daß sich nach der Quellung Stücke mit einem Durchmesser von ca. 1 cm ergeben. Diese Größe ist für die Handhabung und die anschließende Verfaserung besonders geeignet. Im Anschluß an die Quellung und nach Entfernen des Waschwassers wird das gequollene Kollagengranulat in entmineralisiertem Wasser aufgenommen, um es für die anschließende Zerfaserung vorzubereiten. Dabei wird die Wassermenge vorzugsweise so gewählt, daß sich eine 0,1 bis 10 %ige Mischung, bezogen auf das trockene Kollagenmaterial, ergibt. Die Zerfaserung erfolgt vorzugsweise durch Dispergieren unter Rühren, wobei die Kollagenstruktur unter Erzeugung isolierter Kollagenfibrillen aufgebrochen wird. Nach Zugabe des mindestens einen schwerlöslichen Wirkstoffes und ggf. weiterer, weniger schwerlöslicher Wirkstoffe zur Suspension und erneuter Homogenisierung ist diese dann bereit für die anschließende Lyophilisation, ohne daß sie einer weiteren Zwischenbehandlung bedarf.

Die Erfindung wird näher erläutert anhand der nachfolgenden Beschreibung von bevorzugten Ausführungsformen, die auch die näheren Zusammenhänge erkennen lassen sowie anhand eines Ausführungsbeispieles.

<u>Ouellschritt</u>

Das nun vorliegende gereinigte Rinderhaut-Kollagen wird nun in einem ersten Aufarbeitungsschritt mit verdünnter Säure, vorzugsweise 0,1 N Essigsäure, über einen Zeitraum von 6 bis 48 Stunden, vorzugsweise 16 Stunden, gequollen. Durch die saure Quellung wird aus dem harten und spröden Rinderhaut-Kollagen ein elastisches Material, aus dem in einem späteren Schritt mit Hilfe eines mechanischen Vorganges (Schneidevorgang) die Kollagenfibrillen isoliert werden können.

Die Konzentration der Essigsäure während des Quellschrittes wird mit 0,1 N so gewählt, daß sich bei der in einem zweiten Schritt herzustellenden Kollagensuspension ein pH-Wert von 4,0 bis 4,5 einstellt. Liegt der pH-Wert der im zweiten Schritt herzustellenden Kollagensuspension oberhalb 4,8, fallen die Kollagenfasern aus der Suspension aus, und es liegt keine homogene Lösung mehr vor. Liegt der pH-Wert unter 3,5, ist die Kollagenmasse zähflüssig und nicht mehr verarbeitbar.

<u>Spülschritt</u>

Anhand von zwei Versuchsansätzen wird der Einfluß der Spülzyklen auf den pH-Wert bzw. die Essigsäurekonzentration im Spülwasser geprüft.

Die Bestimmung des pH-Wertes erfolgt potentiometrisch. Die Essigsäurekonzentration im jeweiligen Spülwasser wird enzymatisch ermittelt.

Es ergeben sich folgende Meßwerte:

Tabelle 1: pH-Wert und Essigsäurekonzentration bei den Spüllösungen

| Lösung | pH-Wert Versuch 1 | pH-Wert Versuch 2 | Essigsäure-Konz. [mg/l] Versuch 1 | Essigsäure-Konz. [mg/l] Versuch 2 |
|---|---|---|---|---|
| Ansatz 0,1 N Essigsäure | 2,85 | 2,82 | 6,37 | 6,40 |
| 16 h Quellung mit Kollagen | 3,78 | 3,69 | 5,35 | 5,47 |
| 1. Spülung | 3,90 | 3,94 | 0,36 | 0,33 |
| 2. Spülung | 3,85 | 3,85 | 0,16 | 0,12 |
| 3. Spülung | 3,88 | 3,95 | 0,12 | 0,07 |
| 4. Spülung | 3,93 | 4,00 | 0,13 | 0,05 |
| 5. Spülung | 3,98 | 4,05 | 0,08 | 0,05 |

Wie aus der Tabelle zu ersehen ist, reicht 5-maliges Spülen aus, um in der Spüllösung einen pH-Wert von annähernd 4,0 zu erhalten.

Wie bereits erwähnt, wurde die Konzentration der Essigsäure mit 0,1 N beim Quellvorgang so gewählt, daß sich bei der Herstellung der Kollagensuspension ein pH-Wert von 4,0 bis 4,5 einstellt. Während des Quellvorgangs hat sich das Gewicht des Kollagengranulates um den Faktor 4 bis 8 durch entsprechende Wasseraufnahme vergrößert. Es liegt nun als weiches, biegsames, elastisches Material der Größe 1 x 1 x 1 cm vor.

<u>Mechanische Zerkleinerung</u>

Zu dem gequollenen Kollagengranulat wird nun soviel Wasser zugesetzt, daß eine 0,1 bis 10 %ige Aufschlämmung vorliegt. Diese Aufschlämmung wird 2 bis 15 Minuten in einer Hochleistungsdispergiermaschine mit 400 - 1200 U/min homogenisiert. Durch den mechanischen Aufarbeitungsprozeß entsteht ein suspendiertes weitmaschiges Fasergeflecht, das mit der in vivo Anordnung von kollagenen Fasern im Corium keine Gemeinsamkeiten mehr aufweist. Die in vivo kräftigen, sich in allen Richtungen durchflechtenden Kollagenfasern sind aufgebrochen und in kürzere Bruchstücke zerfallen.

Es wurde nun gefunden, daß eine Änderung des pH-Wertes der Kollagensuspension durch Zugabe von 0,1 N Natronlauge bzw. 0,1 N Salzsäure zu folgenden Veränderungen in der Konsistenz der Suspension führt:

| Eingestellter pH-Wert der Kollagensuspension | Konsistenz der Kollagensuspension |
|---|---|
| 3,07 | Gel, breiartig |
| 3,60 | Gel, breiartig |
| 4,03 | gießfähige Suspension |
| 4,30 | gießfähige Suspension* |
| 4,52 | gießfähige Suspension |
| 5,02 | sehr dünnflüssig, Fibrillenaggregation |
| 5,55 | lange Kollagenfibrillen ausgefallen, Trennung von Fasern und Flüssigkeit |

* Hergestellte Suspension ohne Zugabe von Säure und Lauge

Folgende Beobachtungen wurden bei den verschiedenen pH-Wert-Einstellungen an der Kollagensuspension festgestellt:

- Unterhalb eines pH-Wertes von 3,6 wird die Kollagensuspension breiartig. Aber es findet kein Zusammenballen von Fasern zu Faserbündeln statt. Eine Trennung von Fasern und Flüssigkeit ist nicht festzustellen.

- Gibt man zu einer Kollagensuspension vom pH-Wert 3,6 die entsprechende Menge Natronlauge, um einen pH-Wert von 4,3 zu erzielen, so wird aus dem zähflüssigen Gel wieder eine dünnflüssige Suspension. Erhöht man den pH-Wert weiter (5,0), so findet eine Trennung von Fasern und Flüssigkeit statt. Die einzelnen Fasern aggregieren zu Faserbündeln.

Die Untersuchungen belegen, daß in dem bevorzugten pH-Bereich 3,8 bis 4,5 immer eine gießfähige Kollagensuspension vorliegt, die unproblematisch weiterverarbeitet werden kann.

Homogenisierung

Zu der Kollagensuspension wird nun die entsprechende Menge der schwerlöslichen Arzneiform zugegeben.
Unter schwerlöslichen Arzneiformen sind insbesondere Wirkstoffe zu verstehen, die im physiologischen Milieu eine Löslichkeit von < 1 mg/ml haben.
Nach Zugabe der schwerlöslichen Arzneiform wird die wirkstoffhaltige Suspension für 5 bis 30 Minuten unter Rühren mit einem elektrischen Rührwerk mit 20 bis 300 U/min homogenisiert. Die Einbringung der Wirkstoffe erfolgt als wässrige Aufschlämmung oder in Pulverform.
Die Lyophilisation pharmazeutischer Produkte wird seit vielen Jahren praktiziert und zwar in erster Linie mit dem Ziel, instabile Arzneiformen in trockene lagerfähige Formen zu überführen, aus denen sterile Arzneimittel herstellbar sind.
Der wesentlichste Schritt der Lyophilisation ist die Einfrierphase. In dieser Phase wird das Kristallgerüst geschaffen, aus dem die nachfolgende Sublimation erfolgt. Da es sich bei der Kollagensuspension um keine homogene Lösung, sondern um ein Mehrstoffsystem mit festen Bestandteilen handelt, wurden von den üblicherweise durchzuführenden Untersuchungen zur Ermittlung von Einfrierparametern, wie

- Bestimmung des eutektischen Punktes
- Bestimmung der Kollaps-Temperatur
- DSC-Messungen im Tieftemperaturbereich

nur die eutektische Temperatur bestimmt. Untersuchungen von mehreren Kollagensuspensionen aus mehreren Kollagengranulatchargen ergab eine Gefrierpunktserniedrigung auf dem Bereich -2 bis -4 °C für eine Kollagensuspension mit 2 % Massenanteil Kollagen. Die eutektische Temperatur für die Kombination aus Kollagensuspension und schwerlöslicher Arzneiform wird für jede Zusammenstellung vorzugsweise separat ermittelt. Die bei der Bestimmung des eutektischen Punktes erhaltenen Daten bilden die Grundlage der Steuerung der Lyophilisation. Voraussetzung für eine ordnungsgemäße Lyophilisation bei den wirkstoffhaltigen Kollagensuspensionen ist, daß die eutektische Temperatur deutlich unterschritten wird.
Änderungen des pH-Wertes der Kollagensuspension durch Zugabe von Natronlauge bzw. Salzsäure führt zu folgenden Veränderungen in der physikalischen Stabilität des lyophilisierten Vlieses bzw. der erforderlichen Zeit für die vollständige Benetzbarkeit.

Tabelle 3: Einfluß des pH-Wertes der Kollagensuspension auf die physikalische Stabilität der gefriergetrockneten Kollagenmatrix und die Zeit für vollständige Benetzung nach Eintauchen in Wasser

| Eingestellter pH-Wert der suspension | Zeit für vollständige Benetzbarkeit [s] | Phys. Stabilität der lyophilisierten Matrix |
|---|---|---|
| 2,98 | 30,2 | gut |
| 3,40 | 42,7 | gut |
| 3,87 | 27,6 | gut |
| 4,30 | 10,8 | gut |
| 4,45 | 12,6 | gut |
| 4,90 | 7,0 | schlecht* |
| 5,34 | 6,1 | schlecht* |

\* unter einer schlechten Stabilität ist zu verstehen, daß das gefriergetrocknete Vlies nach dem Einlegen in Wasser in einzelne Faseraggregate zerfällt.

Die Untersuchungen zeigen, daß man aus Kollagensuspensionen im pH-Bereich 3,8 bis 4,5 immer Kollagenvliese mit einer guten "Naßstabilität" erhält.

Weitere Schlußfolgerungen sind:

- pH-Werte < 3,8 zeigen eine langsamere Benetzbarkeit als Vliese mit pH-Werten > 3,8.
- Bei eingestellten pH-Werten > 4,5 nimmt die Stabilität der hergestellten Vliese stark ab.
- Die optimale pH-Wert-Einstellung für die Kollagensuspension liegt zwischen pH-Wert 3,8 bis 4,5.
- Liegt der pH-Wert der Kollagensuspension oberhalb 4,8, fallen die Kollagenfasern aus der Kollagensuspension aus und es ist keine homogene Lösung mehr vorhanden.

Die aus Rinderhaut gewonnene wirkstoffhaltige Kollagenmatrix läßt durch den mehrstufigen mechanischen und chemischen Aufarbeitungsprozeß ein weitmaschiges Fasergeflecht erkennen (lichtmikroskopischer Befund), das mit der in vivo-Anordnung von kollagenen Fasern im Corium keine Gemeinsamkeiten mehr aufweist. Die in vivo-kräftigen, sich in allen Richtungen durchflechtenden Kollagenfaserbündel sind aufgebrochen und in kürzere Bruchstücke zerfallen, so daß ein System mit weiten interfibrillären Spalträumen entsteht. Bei der Lyophilisation ist ein grobes Maschenwerk entstanden, das man räumlich betrachtet mit einem Naturschwamm vergleichen kann. Es wird eine gewaltige innere Oberflächenvergrößerung geschaffen, die von kollagenen Fasern gebildet wird.

Elektronenmikroskopische Schnittpräparate zeigen ein Muster aus Kollagenfibrillen zu unterschiedlich dicken Bündeln geordnet, die in alle Richtungen des Raumes verlaufen. Dementsprechend trifft man auf Längs-, Quer- und Tangentialschnitte von Kollagenfibrillen. Längs getroffene Fibrillen besitzen ihre typische Querstreifung. Besonders eindrucksvoll ist das durch die Rasterelektronenmikroskopie sichtbare Oberflächenrelief der lyophilisierten Kollagensuspensionen. Von kräftigen Faserbündeln zweigen nach beiden Seiten dünnere Fasern ab. Dort, wo mehrere dünne Fasern zusammentreffen, entstehen häufig knotenförmige Verdickungen. Daneben kommen mächtige Kollagenfibrillen vor, die seilartig verdrillt sind.

Von zwei verschiedenen Kollagenimplantaten (Matrix mit Clindamycin-Palmitat sowie Matrix mit Clindamycin-Palmitat-Hydrochlorid) schneidet man jeweils 1 cm$^2$ große Stücke zurecht. Die Stücke werden gewogen und das Gewicht protokolliert. Anschließend werden die wirkstoffhaltigen Implantate in Reagenzgläser mit je 5 ml 0.066 M-Phosphatpuffer (pH = 7,4) bzw. Humanserum eingelegt und bei 37 °C im Wasserbad eluiert. Nach 24 Stunden wird das Implantat entnommen und in frische Puffer-Lösung bzw. frisches Serum eingelegt. Die Gesamtelutionszeit beträgt bei 24-stündigem Pufferwechsel 10 Tage. Als biologisches Maß für die Antibiotika-Konzentration dient die Hemmwirkung (Bestimmung des Hemmhofdurchmessers) auf die Testorganismen Staphylococcus aureus ATCC 6538 sowie Micrococcus luteus DSM 348. Dabei wird die Hemmwirkung der Probe mit der Hemmwirkung abgestufter Dosen eines Standards (hier Clindamycin-Hydochlorid) verglichen. Von gleich großen Hemmhöfen bei Proben und Standards kann auf gleich große Antibiotika-Konzentration geschlossen werden.

Die Abgabe der Antibiotika aus den verschiedenen Implantaten kann der Tabelle 4 entnommen werden.

Tabelle 4: Wirkstoffabgabe Clindamycin-Palmitat als Funktion der Elutionszeit

| Tage | Clindamycin-Palmitat in Phosphor-Puffer [μg/Implantat] | Clindamycin-Palmitat-Hydrochlorid in Phosphat-Puffer [ug/Implantat] | Clindamycin-Palmitat in Serum [μg/Implantat] |
|---|---|---|---|
| 1 | 18,3 | 16,0 | 8,0 |
| 2 | 46,1 | 23,8 | 21,2 |
| 3 | 46,1 | 46,1 | 34,7 |
| 4 | 52,7 | 35,4 | 29,1 |
| 5 | 46,1 | 46,1 | 36,1 |
| 6 | 60,1 | 46,1 | 37,8 |
| 7 | 46,1 | 35,4 | 22,1 |
| 8 | 18,3 | 20,8 | 16,5 |
| 9 | 12,3 | 7,2 | 10,8 |
| 10 | 12,3 | 5,5 | 8,1 |

Aus der Tabelle kann man eindeutig erkennen, daßß die Antibiotikaabgabe aus beiden Implantaten auch nach 10 Tagen noch nicht beendet ist. Die abgegebene Menge an Antibiotikum ist bei dem Hydrochlorid des Clindamycin-Palmitat-Esters und dem freien Palmitat-Ester annähernd gleich je Zeiteinheit.

Um zu überprüfen, ob Reste von Antibiotikum auf dem Trägermaterial verblieben sind, wurden die Implantate nach 10 Tagen Elutionszeit auf eine mit Staphylococcus aureus bzw. Micrococcus luteus kontaminierte Agaroberfläche gelegt und bei 37 °C bebrütet. Auch in den Kollagenschwämmen läßt sich nach dieser Zeit noch Antibiotikum nachweisen.

Besteht die Nachfrage nach Implantaten, die einen Wundbereich über einen Zeitraum von 5 bis 15 Tagen schützen sollen, sind die hier beschriebenen Materialien geeignet, diesen Schutz zu gewährleisten. Es liegt hier eine Kombination aus Matrix und Wirkstoff vor, die den Wirkstoff verzögert in geeigneten Konzentrationen über einen Zeitraum von 5 bis 15 Tagen freigeben. Zusätzlich ist die Matrix bis zur vollständigen Resorption durch anhaftendes Antibiotikum vor Keimbesiedlung geschützt. Bei der Untersuchung der Wirkstoffe Clindamycin-Palmitat und Clindamycin-Palmitat-Hydrochlorid wurde nun überraschend gefunden, daß entgegen der in der Literatur publizierten Ergebnisse (Antimicrobial Chemotherapy, herausgegeben von David Green Wood, 3. Auflage, Oxford University Press 1995) auch das mit Palmitinsäure veresterte Clindamycin antibiotische Aktivität zeigt. Bei der Elution im wässrigen Puffersystem konte überra-schenderweise antibiotische Wirksamkeit über einen Zeitraum von mindestens 11 Tagen nachgewiesen werden. Die Veresterung der OH-Gruppe von Clindamycin mit Palmitinsäure setzt zwar die Löslichkeit von Clindamycin drastisch herab (siehe Tabelle 5), führt aber nicht zu einer Inaktivierung des Moleküls bezüglich seiner antibiotischen Wirksamkeit. Das heißt, Clindamycin-Palmitat muß nicht erst enzymatisch durch esterasen in das wirksame Molekül Clindamycin gespalten werden. Das ist bedeutsam, da dadurch der Einsatz einer Clindamycin-Palmitat-haltigen Kollagen-Matrix nicht an das Vorhandensein von Enzymen gekoppelt ist.

Tabelle 5: Unregelmäßiges Löslichkeitsverhalten der Palmitat-Ester von Lincomycin und Clindamycin*

| pH | Lincomycin-Palmitat-Hydrochlorid [mg/ml] | pH | Clindamycin-Palmitat-Hydrochlorid [mg/ml] |
|---|---|---|---|
| 2,3 | 0,124 | 3,7 | 53,2 |
| - | - | 5,8 | < 0,001 |
| 7,7 | 0,0249 | 7,4 | < 0,0002 |

Literatur: E.L. Rowe, Journal of Pharmaceutical Sciences, Vol. 68, No. 10 (1979), Seiten 1292 - 1296.

Ausführungsbeispiel

1. Quellschritt

1823,1 g Kollagengranulat (Wassergehalt 15,0 % mittlere Granulatgröße 4 bis 6 mm) werden nach Quellung in 36 l 0,1 N Essigsäure über 16 Stunden 5 mal mit 5 l Wasser für Injektionszwecke gespült. Der pH-Wert der Spüllösung muß nach der fünften Spülung > 4,0 sein. Andernfalls muß weitergespült werden.

2. Mechanische Zerkleinerung

Anschließend wird mit Wasser für Injektionszwecke auf ein Gesamtgewicht von 90,0 kg unter Berücksichtigung der später zuzugebenden Menge an schwerlöslicher Arzneiform aufgefüllt. Die oben angeführte Aufschlämmung wird für 2 Minuten in einer Hochleistungs-Dispergiermaschine mit 680 U/min homogenisiert. Der pH-Wert der homogenisierten Masse wird überprüft.

3. Homogenisierung

Zu der Kollagensuspension werden nun 224,48 g Clindamycin-Palmitat-Hydrochlorid (entsprechend 136,26 g Clindamycin-Base), suspendiert in 5 1 0,1 N-Acetat-Puffer vom pH = 3,6 bis 3,8, zugegeben. Die wirkstoffhaltige suspension wird erneut für 15 Minuten mit einem elektrischen Rührwerk mit 150 U/min homogenisiert. Die Gesamtmasse der wirkstoffhaltigen Suspension beträgt jetzt 90,0 kg.

4. Lyophilisation

Zur Bestimmung des Aufgießgewichtes auf die Gefriertrocknungsschalen (Größe 45,6 x 45,6 cm) wird die erforderliche Menge an wirkstoffhaltiger Kollagensuspension pro Vlies in Relation zu der verfügbaren Fläche je Gefriertrocknungsschale gesetzt.

Die Plattengröße der Kollagenvliese nach der Gefriertrocknung beträgt 43,5 cm x 43,5 cm 1892,15 cm$^2$. Ein wirkstoffhaltiges Kollagenvlies der Größe 5 cx 8 cm = 40,0 cm$^2$ soll 35 mg Clindamycin und 400 mg wasserfreies Kollagen enthalten.

$$\text{Clindamycin-Base/Schale} = \frac{1892,25 \ \text{cm}^2}{40 \ \text{cm}^2} \times 0,035 \ \text{g} = 1,6557 \ \text{g}$$

$$\text{Es sind deshalb:} \quad \frac{1,6557 \ \text{g}}{136,26 \ \text{g}} \times 90000,0 \ \text{g} = 1093,6 \ \text{g Kollagen-suspension}$$

auf jede Gefriertrocknungsschale aufzugießen.

Die gefüllten Gefriertrocknungsschalen werden in die Gefriertrocknungsanlage eingebracht. Folgende Vorgaben gelten für den Gefriertrocknungsprozeß:

| | | |
|---|---|---|
| Beladen der Anlage: | +7 °C | |
| Produkttemperatur beim Einfrieren: Kammerinnendruck beim Einfrieren: | +7 °C 10$^3$ mbar | -45 °C |
| Produkttemperatur bei der Haupttrocknung: Kammerinnendruck bei der Haupttrocknung: | -45 °C 0,9 mbar | +38 °C |
| Produkttemperatur beim Nachtrocknen: Kammerinnendruck beim Nachtrocknen: | +38 °C 0,03 mbar | +21 °C |

5. Sterilisation

Die Sterilisation erfolgt durch Strahlensterilisation mit 25 kGy oder Ethylenoxid.

**Patentansprüche**

**1.** Wirkstoffmatrix in Form eines biologisch resorbierbaren porösen Vlieses aus unvernetzten Kollagenfibrillen in lyophilisierter Form mit einer retardierten Freigabe von Wirkstoffen über mehr als 10 Tage und enthaltend in homogener Verteilung 3 bis 30 Gew.-% mindestens eines in Wasser und Körperflüssigkeiten schwerlöslichen Wirkstoffs, wobei mindestens ein Wirkstoff in physiologischem Milieu eine Löslichkeit von < 10 mg/ml aufweist, wobei die Wirkstoffmatrix eine offene Struktur aufweist, Nassstabilität besitzt und außer den Kollagenfibrillen als Trägerstruktur und dem mindestens einen Wirkstoff im Wesentlichen frei von weiteren Bestandteilen ist und im Wesentlichen salzfrei ist, und worin der mindestens eine Wirkstoff nach Implantation retardiert und in geeigneter Konzentration freigesetzt wird.

2. Biologisch abbaubare Wirkstoffmatrix in Form eines offenporigen Vlieses oder Schwammes aus unvernetzten resorbierbaren Kollagenfibrillen, insbesondere zur Herstellung der Wirkstoffmatrix nach Anspruch 1, **dadurch** erhältlich, dass man Stücke aus gereinigter, entfetteter und getrockneter Haut in verdünnten wässrigen Lösungen organischer Säuren quellen lässt, bis ein elastisches Material entsteht, die gequollenen Stücke unter Vergrößerung des pH-Wertes mehrfach mit wässrigen Medien, insbesondere entmineralisiertem Wasser, spült, die gespülten Stücke zur Bildung einer Suspension von Kollagenfibrillen mechanisch zerfasert, die einen pH-Wert von > 3,5 bis < 4,8 aufweisende, gießfähige Kollagensuspension mit mindestens einem Wirkstoff in feinverteilter Form versetzt und homogenisiert, wobei mindestens ein Wirkstoff in physiologischem Milieu schwerlöslich ist und eine Löslichkeit < 10 mg/ml aufweist, und die wirkstoffhaltige Suspension anschließend zu dem Vlies bzw. Schwamm lyophilisiert.

3. Wirkstoffmatrix nach Anspruch 1 oder 2, **dadurch gekennzeichnet, dass** sie eine Schichtdicke von 0,5 mm bis 15 mm, insbesondere 2 mm bis 5 mm besitzt.

4. Wirkstoffmatrix nach einem der Ansprüche 1 bis 3, **dadurch gekennzeichnet, dass** sie eine Dichte von 12 mg/cm$^3$ bis 180 mg/cm$^3$ besitzt.

5. Wirkstoffmatrix nach einem der vorhergehenden Ansprüche, **dadurch gekennzeichnet, dass** sie ein Porenvolumen von 60 bis 80 % des gesamten Volumens besitzt.

6. Wirkstoffmatrix nach einem der vorhergehenden Ansprüche, **dadurch gekennzeichnet, dass** sie eine mittlere Porengröße im Bereich von 20 $\mu$m bis 150 $\mu$m besitzt.

7. Wirkstoffmatrix nach einem der vorhergehenden Ansprüche, **dadurch gekennzeichnet, dass** sie eine Luftdurchlässigkeit von 2500 bis 5000 ml/cm$^2$/min, insbesondere 2700 bis 3400 ml/cm$^2$/min, bei einer Schichtdicke von 4,2 mm besitzt.

8. Wirkstoffmatrix nach einem der vorhergehenden Ansprüche, **dadurch gekennzeichnet, dass** der mindestens eine schwerlösliche Wirkstoff ein Arzneimittel, insbesondere ein Antibiotikum, ist.

9. Wirkstoffmatrix nach einem der vorhergehenden Ansprüche, **dadurch gekennzeichnet, dass** als Antibiotikum Aminoglycosid-Antibiotika, insbesondere Clindamycin-Palmitat, Clindamycin-Palmitat-Hydrochlorid und/oder Gentamicin-Crobefat vorgesehen sind.

10. Wirkstoffmatrix nach einem der vorhergehenden Ansprüche, **dadurch gekennzeichnet, dass** sie zusätzlich zu dem mindestens einen schwerlöslichen Wirkstoff mindestens einen wenigerschwer- oder leichtlöslichen Wirkstoff enthält.

11. Verfahren zur Herstellung einer biologisch abbaubaren Wirkstoffmatrix in Form eines offenporigen Vlieses oder Schwammes aus unvernetzten resorbierbaren Kollagenfibrillen, insbesondere zur Herstellung der Wirkstoffmatrix nach einem der vorhergehenden Ansprüche, **dadurch gekennzeichnet, dass** man Stücke aus gereinigter, entfetteter und getrockneter Haut in verdünnten wässrigen Lösungen organischer Säuren quellen lässt, bis ein elastisches Material entsteht, die gequollenen Stücke unter Vergrößerung des pH-Wertes mehrfach mit wässrigen Medien, insbesondere entmineralisiertem Wasser, spült, die gespülten Stücke zur Bildung einer Suspension von Kollagenfibrillen mechanisch zerfasert, die einen pH-Wert von > 3,5 bis < 4,8 aufweisende, gießfähige Kollagensuspension mit mindestens einem schwerlöslichen Wirkstoff in feinverteilter Form versetzt und homogenisiert und die wirkstoffhaltige Suspension anschließend zu dem Vlies bzw. Schwamm lyophilisiert.

12. Verfahren nach Anspruch 11, **dadurch gekennzeichnet, dass** die Konzentration der für die Quellung verwendeten organischen Säure und die Anzahl der Spülungen so gewählt und aufeinander eingestellt werden, dass nach der Spülung und Zerfaserung ohne vorherige pH-Korrektur eine Kollagensuspension mit einem pH-Wert von > 3,5 bis < 4,8, insbesondere von 4 bis 4,5 erhalten wird.

**13.** Verfahren nach Anspruch 11 oder 12, **dadurch gekennzeichnet, dass** die Spülung mindestens zwei, insbesondere mindestens fünf Spülzyklen, umfasst.

**14.** Verfahren nach Anspruch 11 bis 13, **dadurch gekennzeichnet, dass** die Quellung während 5 bis 60 Stunden, insbesondere 6 bis 48 Stunden, durchgeführt wird.

**15.** Verfahren nach einem der Ansprüche 11 bis 13, **dadurch gekennzeichnet, dass** zur Quellung eine Säurelösung mit einer Säurekonzentration von 0,01 bis 2 N, insbesondere 0,05 bis 0,5 N, verwendet wird.

**16.** Verfahren nach einem der Ansprüche 11 bis 15, **dadurch gekennzeichnet, dass** die Hautstücke in der organischen Säure auf das 3- bis 10flache, insbesondere 4- bis 8fache, ihres Gewichtes aufgequollen werden.

**17.** Verfahren nach einem der Ansprüche 11 bis 16, **dadurch gekennzeichnet, dass** das gequollene Kollagengranulat nach dem Spülen und Entfernen des Spülwassers durch Wasserzusatz in eine 0,1 bis 10 %ige Mischung, bezogen auf das Gewicht des trockenen Kollagenmaterials, überführt wird und diese Mischung durch Dispergieren zu der Kollagensuspension homogenisiert wird, wobei der Faserverbund der Kollagenfibrillen aufgebrochen wird.

**18.** Verfahren nach einem der Ansprüche 11 bis 17, **dadurch gekennzeichnet, dass** der mindestens eine schwerlösliche Wirkstoff in feinverteilter Form, insbesondere suspendiert in einem wässrigen Medium zugegeben wird.

**19.** Verfahren nach einem der Ansprüche 11 bis 18, **dadurch gekennzeichnet, dass** die Suspension der Kollagenfibrillen nach der Zugabe des mindestens einen schwerlöslichen Wirkstoffes zur gleichmäßigen Verteilung des mindestens einen Wirkstoffes in der Suspension homogenisiert wird.

**20.** Verfahren nach einem der Ansprüche 11 bis 19, **dadurch gekennzeichnet, dass** außer dem mindestens einen schwerlöslichen Wirkstoff mindestens ein weniger schwerlöslicher Wirkstoff, vorzugsweise gleicher Wirkungsrichtung, zugegeben wird.

**21.** Verfahren nach einem der Ansprüche 11 bis 20, **dadurch gekennzeichnet, dass** die homogenisierte, wirkstoffhaltige Kollagensuspension ohne weitere Zwischenbehandlung zu insbesondere flächenhaften Vliesen oder Schwämmen lyophilisiert wird.

**22.** Verwendung der Wirkstoffmatrix nach einem der Ansprüche 1 bis 10 zur Herstellung eines implantierbaren und vollständig resorbierbaren Depots für Wirkstoffe mit einer retardierten Wirkstoffabgabe.

**Claims**

**1.** Active-ingredient matrix in the form of a bioabsorbable porous web of uncrosslinked collagen fibrils in lyophilized form providing a controlled release of active ingredients over more than 10 days and containing in homogeneous distribution 3% to 30% by weight of at least one active ingredient sparingly soluble in water and bodily fluids, wherein at least one active ingredient has a solubility of < 10 mg/ml in physiological medium, wherein the active-ingredient matrix displays an open structure, has wet stability and aside from the collagen fibrils as carrier structure and the at least one active ingredient is essentially free of further constituents and is essentially salt-free, and wherein the release of the at least one active ingredient after implantation takes place in a controlled manner and in a suitable concentration.

**2.** Biodegradable active-ingredient matrix in the form of an open-pore web or sponge of uncrosslinked absorbable collagen fibrils, particularly for forming the active-ingredient matrix according to Claim 1, obtainable by allowing pieces of cleaned, defatted and dried skin to swell in dilute aqueous solutions of organic acids until an elastic material has formed, rinsing the swollen pieces repeatedly with aqueous media, particularly demineralized water, to increase the pH, mechanically defiberizing the rinsed pieces to form a suspension of collagen fibrils, admixing the pourable collagen suspension which has a pH of > 3.5 to < 4.8 with at least one active ingredient in finely divided form and homogenizing the admixture, at least one active ingredient being sparingly soluble in physiological medium and displaying a solubility < 10 mg/ml in physiological medium, and subsequently lyophilizing the active ingredient-containing suspension to form the web or sponge.

**3.** Active-ingredient matrix according to Claim 1 or 2, **characterized in that** it has a layer thickness of 0.5 mm to 15 mm, in particular 2 mm to 5 mm.

4. Active-ingredient matrix according to any one of Claims 1 to 3, **characterized in that** it has a density of 12 mg/cm$^3$ to 180 mg/cm$^3$.

5. Active-ingredient matrix according to any one of the preceding claims, **characterized in that** it has a pore volume of 60 to 80% of the entire volume.

6. Active-ingredient matrix according to any one of the preceding claims, **characterized in that** it has an average pore size in the range from 20 $\mu$m to 150 $\mu$m.

7. Active-ingredient matrix according to any one of the preceding claims, **characterized in that** it has an air permeability of 2500 to 5000 ml/cm$^2$/min, in particular 2700 to 3400 ml/cm$^2$/min, at a layer thickness of 4.2 mm.

8. Active-ingredient matrix according to any one of the preceding claims, **characterized in that** the at least one sparingly soluble active ingredient is a drug, more particularly an antibiotic.

9. Active-ingredient matrix according to any one of the preceding claims, **characterized in that** aminoglycoside antibiotics are provided as antibiotic, more particularly clindamycin palmitate, clindamycin palmitate hydrochloride and/or gentamicin crobefat.

10. Active-ingredient matrix according to any one of the preceding claims, **characterized in that** in addition to the at least one sparingly soluble active ingredient it contains at least one less sparingly or readily soluble active ingredient.

11. Process for forming a biodegradable active-ingredient matrix in the form of an open-pore web or sponge of uncrosslinked absorbable collagen fibrils, particularly for forming the active-ingredient matrix according to any one of the preceding claims, **characterized in that** it comprises allowing pieces of cleaned, defatted and dried skin to swell in dilute aqueous solutions of organic acids until an elastic material has formed, rinsing the swollen pieces repeatedly with aqueous media, particularly demineralized water, to raise the pH, mechanically defiberizing the rinsed pieces to form a suspension of collagen fibrils, admixing the pourable collagen suspension which has a pH of > 3.5 to < 4.8 with at least one sparingly soluble active ingredient in finely divided form and homogenizing the admixture and subsequently lyophilizing the active ingredient-containing suspension to form the web or sponge.

12. Process according to Claim 11, **characterized in that** the concentration of the organic acid used for the swelling and the number of rinses are chosen and adjusted relative to each other such that after the rinsing and defiberization without prior pH correction a collagen suspension having a pH of > 3.5 to < 4.8, more particularly of 4 to 4.5 is obtained.

13. Process according to Claim 11 or 12, **characterized in that** the rinsing comprises at least two, more particularly at least five rinsing cycles.

14. Process according to Claims 11 to 13, **characterized in that** the swelling is carried out during 5 to 60 hours, more particularly 6 to 48 hours.

15. Process according to any one of Claims 11 to 13, **characterized in that** the swelling utilizes an acid solution having an acid concentration of 0.01 to 2 N, more particularly 0.05 to 0.5 N.

16. Process according to any one of Claims 11 to 15, **characterized in that** the skin pieces are swollen in the organic acid to 3 to 10 times, more particularly 4 to 8 times their weight.

17. Process according to any one of Claims 11 to 16, **characterized in that** the swollen granular collagen after the rinsing and removing of the rinse water is converted into a 0.1% to 10% by weight mixture, based on the weight of the dry collagen material, by addition of water and this mixture is homogenized by dispersing to form the collagen suspension by disrupting the fibrous assembly of the collagen fibrils.

18. Process according to any one of Claims 11 to 17, **characterized in that** the at least one sparingly soluble active ingredient is added in finely divided form, more particularly as a suspension in an aqueous medium.

19. Process according to any one of Claims 11 to 18, **characterized in that** the suspension of the collagen fibrils after the addition of the at least one sparingly soluble active ingredient is homogenized to uniformly distribute the at least one active ingredient in the suspension.

20. Process according to any one of Claims 11 to 19, **characterized in that** in addition to the at least one sparingly soluble active ingredient at least one less sparingly soluble active ingredient, preferably of the same direction of action, is added.

21. Process according to any one of Claims 11 to 20, **characterized in that** the homogenized, active ingredient-containing collagen suspension is lyophilized without further intervening treatment to form specifically sheetlike webs or sponges.

22. Use of the active-ingredient matrix according to any one of Claims 1 to 10 in the manufacture of an implantable and fully absorbable depot for active ingredients providing a controlled delivery of active ingredient.

**Revendications**

1. Matrice pour substance active, sous forme d'un non-tissé poreux biologiquement résorbable à base de fibrilles de collagène non réticulées sous forme lyophilisée, présentant une libération retardée de substances actives pendant plus de 10 jours et contenant en répartition homogène 3 à 30 % en poids d'au moins une substance active difficilement soluble dans l'eau et les liquides corporels, au moins une substance active ayant en milieu physiologique une solubilité de < 10 mg/ml, la matrice pour substance active présentant une structure ouverte, possédant une stabilité vis-à-vis de l'humidité et étant pratiquement exempte de sels et pratiquement exempte d'autres composants, hormis les fibrilles de collagène, en tant que structure porteuse, et dans laquelle ladite au moins une substance active après implantation est libérée de façon retardée et à une concentration appropriée.

2. Matrice pour substance active, biologiquement dégradable, sous forme d'une éponge ou d'un non-tissé à pores ouverts, à base de fibrilles de collagène non réticulées, résorbables, en particulier pour la production de la matrice pour substance active selon la revendication 1, pouvant être obtenue en faisant gonfler des fragments de peau nettoyée, dégraissée et séchée, dans des solutions aqueuses diluées d'acides organiques, jusqu'à l'obtention d'une matière élastique, puis en rinçant plusieurs fois avec des milieux aqueux, en particulier de l'eau déminéralisée, les fragments gonflés, avec élévation du pH, et en séparant mécaniquement les fibres des fragments rincés, pour la formation d'une suspension de fibrilles de collagène, puis en ajoutant à la suspension fluide de collagène, présentant un pH de > 3,5 à < 4,8, au moins une substance active sous forme finement divisée et en homogénéisant, au moins une substance active étant difficilement soluble en milieu physiologique et présentant une solubilité < 10 mg/ml, et ensuite en lyophilisant la suspension contenant la/les substance(s) active(s) pour obtenir le non-tissé ou l'éponge.

3. Matrice pour substance active selon la revendication 1 ou 2, **caractérisée en ce qu'**elle présente une épaisseur de couche de 0,5 mm à 15 mm, en particulier de 2 mm à 5 mm.

4. Matrice pour substance active selon l'une quelconque des revendications 1 à 3, **caractérisée en ce qu'**elle présente une densité de 12 mg/cm$^3$ à 180 mg/cm$^3$.

5. Matrice pour substance active selon l'une quelconque des revendications précédentes, **caractérisée en ce qu'**elle présente un volume de pores de 60 à 80 % du volume total.

6. Matrice pour substance active selon l'une quelconque des revendications précédentes, **caractérisée en ce qu'**elle présente une taille moyenne de pore dans la plage de 20 $\mu$m à 150 $\mu$m.

7. Matrice pour substance active selon l'une quelconque des revendications précédentes, **caractérisée en ce qu'**elle présente une perméabilité à l'air de 2 500 à 5 000 ml/cm$^2$/min, en particulier de 2 700 à 3 400 ml/cm$^2$/min, pour une épaisseur de couche de 4,2 mm.

8. Matrice pour substance active selon l'une quelconque des revendications précédentes, **caractérisée en ce que** ladite au moins une substance active difficilement soluble est un médicament, en particulier un antibiotique.

9. Matrice pour substance active selon l'une quelconque des revendications précédentes, **caractérisée en ce qu'**on prévoit comme antibiotique des antibiotiques aminoglycoside, en particulier le palmitate de clindamycine, le palmitate de clindamycine chlorhydrate et/ou le crobéfate de gentamicine.

**10.** Matrice pour substance active selon l'une quelconque des revendications précédentes, **caractérisée en ce qu'**outre ladite au moins une substance active difficilement soluble elle contient au moins une substance active aisément ou moins difficilement soluble.

**11.** Procédé pour la production d'une matrice biologiquement dégradable pour substance active, sous forme d'un non-tissé ou d'une éponge à pores ouverts, à base de fibrilles de collagène non réticulées, résorbables, en particulier pour la production de la matrice pour substance active selon l'une quelconque des revendications précédentes, **caractérisé en ce qu'**on fait gonfler des fragments de peau nettoyée, dégraissée et séchée, dans des solutions aqueuses diluées d'acides organiques, jusqu'à l'obtention d'une matière élastique, on rince plusieurs fois avec des milieux aqueux, en particulier de l'eau déminéralisée, les fragments gonflés, avec élévation du pH, on sépare mécaniquement les fibres des fragments rincés, pour la formation d'une suspension de fibrilles de collagène, on ajoute à la suspension fluide de collagène, présentant un pH de > 3,5 à < 4,8, au moins une substance active difficilement soluble, sous forme finement divisée et on homogénéise et ensuite on lyophilise la suspension contenant la/les substance(s) active(s) pour obtenir le non-tissé ou l'éponge.

**12.** Procédé selon la revendication 11, **caractérisé en ce que** la concentration de l'acide organique utilisé pour le gonflement et le nombre des rinçages sont choisis et adaptés l'un à l'autre de manière à obtenir après le rinçage et la séparation des fibres, sans correction préliminaire du pH, une suspension de collagène ayant un pH de > 3,5 à < 4,8, en particulier de 4 à 4,5.

**13.** Procédé selon la revendication 11 ou 12, **caractérisé en ce que** le rinçage comprend au moins deux, en particulier au moins cinq cycles de rinçage.

**14.** Procédé selon l'une quelconque des revendications 11 à 13, **caractérisé en ce que** le gonflement est effectué pendant 5 à 60 heures, en particulier 6 à 48 heures.

**15.** Procédé selon l'une quelconque des revendications 11 à 13, **caractérisé en ce que** pour le gonflement on utilise une solution d'acide ayant une concentration d'acide de 0,01 à 2 N, en particulier de 0,05 à 0,5 N.

**16.** Procédé selon l'une quelconque des revendications 11 à 15, **caractérisé en ce que** les fragments de peau sont gonflés dans l'acide organique jusqu'au triple au décuple, en particulier jusqu'au quadruple à l'octuple, de leur poids.

**17.** Procédé selon l'une quelconque des revendications 11 à 16, **caractérisé en ce qu'**après le rinçage et l'élimination de l'eau de rinçage, on convertit par addition d'eau le produit granulé de collagène gonflé en un mélange à 0, 1-10 %, par rapport au poids de la matière collagène sèche et on homogénéise ce mélange par dispersion en la suspension de collagène, l'assemblage en fibres des fibrilles de collagène étant rompu.

**18.** Procédé selon l'une quelconque des revendications 11 à 17, **caractérisé en ce que** ladite au moins une substance active difficilement soluble est ajoutée sous forme finement divisée, en particulier en suspension dans un milieu aqueux.

**19.** Procédé selon l'une quelconque des revendications 11 à 18, **caractérisé en ce qu'**après l'addition de ladite au moins une substance active difficilement soluble, la suspension des fibrilles de collagène est homogénéisée pour la répartition homogène de ladite au moins une substance active dans la suspension.

**20.** Procédé selon l'une quelconque des revendications 11 à 19, **caractérisé en ce que**, outre ladite au moins une substance active difficilement soluble on ajoute au moins une substance active moins difficilement soluble, de préférence à même orientation d'action.

**21.** Procédé selon l'une quelconque des revendications 11 à 20, **caractérisé en ce que** la suspension de collagène homogénéisée, contenant une/des substance(s) active(s), est lyophilisée sans autre traitement intermédiaire, en éponges ou non-tissés en particulier plan(e)s.

**22.** Utilisation de la matrice pour substance active selon l'une quelconque des revendications 1 à 10, pour la fabrication d'une préparation retard implantable et totalement résorbable pour des substances actives avec une libération retardée de la substance active.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3212412 C2
- DE 3124981 A1
- EP 0360180 B1
- EP 0137297 A
- EP 0209726 A, Beutler/Eblingerl Lindner
- DE OS3203957 A, Eckmayer
- US PS3157524 A, Artandi
- DE OS3315678 A, Cioca
- DE 1811290, Chvapil
- EP 0173186 A1

**In der Beschreibung aufgeführte Nicht-Patentliteratur**

- **von Wachol-Drewek ; Pfeiffer ; Scholl.** *Biomaterials,* 1996, vol. 17, 1733-1738
- Antimicrobial Chemotherapy. Oxford University Press, 1995
- **E.L. Rowe.** *Journal of Pharmaceutical Sciences,* 1979, vol. 68 (10), 1292-1296